# EUROPEAN PATENT SPECIFICATION

(11) **EP 2 555 799 B1**
(45) Date of publication and mention of the grant of the patent: **07.03.2018**
(21) Application number: 10849329.7
(22) Date of filing: 08.06.2010
(51) Int. Cl.: A23L 5/30, A23L 3/005, A23C 3/07

(54) **LIGHT ENERGY-INDUCED STABILITY OF BIOMATERIALS**
LICHTENERGIEINDUZIERTE STABILITÄT VON BIOMATERIALIEN
STABILITÉ DE BIOMATÉRIAUX INDUITE PAR L'ÉNERGIE LUMINEUSE

(30) Priority: 08.04.2010 IN 401KO2010
(43) Date of publication of application: 13.02.2013
(73) Proprietor: University Of Calcutta, Kolkata 700 073 West Bengal (IN)
(72) Inventor: SINGHA, Santiswarup, Ramchandrapur 721652 (IN); DASGUPTA, Anjan, Kr., Kolkata 700003 (IN)
(74) Representative: Grünecker Patent- und Rechtsanwälte PartG mbB
(86) International application number: PCT/IB2010/001373
(87) International publication number: WO 2011/124944

(56) References cited:
- WO-A1-98/38876
- WO-A1-03/103390
- US-A- 5 766 600
- US-A1- 2001 024 830
- 'Spectroscopy: Principles, Theory, Techniques and Applications', [Online] 30 May 2009, Retrieved from the Internet: <URL:http://novelresearchinstitute.org/libr ary/SpectroscopyOvrview.pdf> [retrieved on 2010-10-07]

## Description

### TECHNICAL FIELD

This disclosure relates generally to methods for enhancing the stability of labile biomolecules, such as proteins.

### BACKGROUND

The following description is provided to assist the understanding of the reader. None of the information provided or references cited is admitted to be prior art.

Changes in the structure of biomolecules, such as proteins, can be caused by a variety of factors. For example, when many biomolecules are exposed to increasing temperature, loss of solubility or activity may occur over a fairly narrow range. Depending upon the biomolecule studied and the severity of the heating, these changes may or may not be reversible. For example, as the temperature of a protein is increased, a number of bonds in the protein molecule are weakened. The first affected are the long range interactions that are necessary for the maintenance of tertiary structure. As these bonds are weakened or broken, the protein obtains a more flexible structure and the groups are exposed to solvent. If heating ceases at this stage, then the protein should be able to readily refold to the native structure. As heating continues, some of the cooperative hydrogen bonds that stabilize the helical structure will begin to break. As these bonds are broken, water can interact with and form new hydrogen bonds with the amide nitrogen and carbonyl oxygens of the peptide bonds. As the helical structure is broken, hydrophobic groups are exposed to the solvent.

The effect of exposure of new hydrogen bonding groups and of hydrophobic groups is to increase the amount of water bound by the protein molecules. The unfolding that occurs increases the hydrodynamic radius of the molecule causing the viscosity of the solution to increase. The net result will be an attempt by the protein to minimize its free energy by burying as many hydrophobic groups while exposing as many polar groups as possible to the solvent. Upon cooling, the structures obtained by the aggregated proteins may not be those of lowest possible free energy, but kinetic barriers will prevent them from returning to the native structure. Exposure of most proteins to high temperatures results in irreversible denaturation.

Chaperones are proteins that assist in the non-covalent folding and assembly of other macromolecular structures, but do not occur in these structures when the latter are performing their normal biological functions. One major function of chaperones is to prevent both newly synthesized polypeptide chains and assembled subunits from aggregating into non-functional structures. It is for this reason that many chaperones, but by no means all, are also heat shock proteins because the tendency to aggregate increases as proteins are denatured by stress.
WO 98/38876 relates to a method of inhibiting growth of microorganisms and improving storage stability of milk by laser radiation.

### SUMMARY

In one aspect, the present disclosure provides a method for stabilizing biomolecules according to claim 1. In one embodiment, the one or more labile biomolecules are exposed to light energy in the presence of a denaturant.

In one embodiment, the denaturant is an agent capable of inducing a change in the secondary, tertiary, or quaternary structure of a labile biomolecule. In one embodiment, the denaturant is heat. In one embodiment, the heat includes heating the labile biomolecule above a physiological temperature. In one embodiment, the heat denaturant includes heating the labile biomolecule to a temperature from about 40°C to about 100°C. In one embodiment, the denaturant is a chemical denaturant that disrupts the secondary, tertiary, or quaternary structure of a labile biomolecule. In one embodiment, the chemical denaturant is a reducing agent, such as dithiothreitol.

In one embodiment, the proteins undergo aggregation and/or unfolding in the absence of an effective amount of light energy. In one embodiment, aggregation of the proteins is reduced from about 10% to about 60% compared to the aggregation of the one or more proteins not exposed to the light energy. In one embodiment, the proteins are selected from the group consisting of: hemoglobin, insulin and citrate synthase.

In one embodiment, the one or more labile proteins in aqueous solution are in a material selected from the group consisting of: a food, a drink, a therapeutic agent, an implant, and combinations thereof. In one embodiment, the food is an egg preparation. In one embodiment, the drink is milk.

In one embodiment, the light energy is red wavelength laser radiation. In one embodiment, the wavelength of the light energy is about 630 ± 20 nm. In one embodiment, the light energy has a power density of less than about 1.8 watts/cm². In one embodiment, the light energy produces a NIR emission at about about 900 to about 1000 nm.

In one aspect, the present disclosure provides a method for stabilizing biomolecules during pasteurization, the method comprising: exposing a material having one or more labile biomolecules in aqueous solution to an effective amount of light energy to at least partially stabilize the one or more labile biomolecules in the solution; and simultaneously pasteurizing the material. In one embodiment, the material comprises an industrial enzyme, a recombinant protein, a food, a drink, a therapeutic agent, a medical device, or combinations thereof.

The foregoing summary is illustrative only and is not intended to be in any way limiting. In addition to the illustrative aspects, embodiments, and features described above, further aspects, embodiments, and features will become apparent by reference to the following drawings and the detailed description.

### BRIEF DESCRIPTION OF THE FIGURES

FIG. 1 depicts a block diagram of an illustrative embodiment of a biomolecule stabilization system.
FIG. 2 shows an illustrative embodiment of a system for the treatment of a biomaterial in the presence and optionally the absence of light energy (e.g., laser light).
FIG. 3 shows a chart illustrating the extent of aggregation in the presence and absence of laser with different exemplary proteins. The darker bars represent aggregation in the absence of a laser (control samples) whereas the lighter bars represent aggregation of test samples in the presence of a laser.
FIG. 4 is an illustrative dynamic light scattering pattern (DLS) of hemoglobin in the presence and absence of red laser light.
FIG. 5 shows the extent of aggregation of citrate synthase in the presence of low power (5 mW) and high power (15 mW) red laser light. The results show a decrease in the aggregation of citrate synthase upon exposure to low power (5 mW) red laser, but an increase in aggregation of citrate synthase upon exposure to high power (15 mW) red laser.

### DETAILED DESCRIPTION

In the following detailed description, reference may be made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented here.

As used herein, unless otherwise stated, the singular forms "a," "an," and "the" include plural reference. Thus, for example, a reference to "a protein" includes a plurality of protein molecules.

As used herein, the term "about" will be understood by persons of ordinary skill in the art and will vary to some extent depending upon the context in which it is used. If there are uses of the term which are not clear to persons of ordinary skill in the art, given the context in which it is used, the term "about" in reference to quantitative values will mean up to plus or minus 10% of the enumerated value.

As used herein, the term "aggregation" refers to a process whereby biomolecules, such as polypeptides, stably associate with each other to form a multimeric, insoluble complex, which does not disassociate under physiological conditions.

As used herein, the term "laser" refers to electromagnetic radiation of any frequency that is amplified by stimulated emission of radiation. A laser also refers to a device that emits electromagnetic radiation through a process called stimulated emission. Laser light is usually spatially coherent, which means that the light either is emitted in a narrow, low-divergence beam, or can be converted into one with the help of optical components such as lenses. As used herein, the term "red wavelength laser radiation" refers to laser radiation having wavelengths in the range from about 600 to about 700 nm.

As generally used herein, the term "labile" refers to the property of a molecule or bond to undergo chemical, physical, or biological change, degradation, or disruption.

As used herein, the term "labile biomolecules" refers to a biomolecule which loses a substantial amount of activity or structure when either heated to elevated temperatures, such as temperatures greater than physiological temperatures, or exposed to a chemical denaturant. In the former case, the biomolecule can also be referred to as a "thermally labile biomolecule", while in the latter case, the biomolecule can be referred to as a "chemically labile biomolecule". Examples of labile biomolecules include proteins, polypeptides, nucleic acids, and polysaccharides. Molecules of these types often exist under physiological conditions in conformations essential to activity, and, upon heating, undergo a conformational change. The active conformations can be stabilized by interactions such as hydrogen bonds and salt bridges, which can be disrupted when the molecule is dissolved in a nonaqueous solvent, such as dimethylsulfoxide. The stabilization methods described herein are particularly advantageous for labile biomolecules, because it enables treatment of the labile biomolecules with chemicals or elevated temperatures, and yet, the biomolecules retain their activity or structure.

The term "light energy" as used herein refers to any type of electromagnetic radiation or energy, whether comprised of a narrow, discrete frequency or multiple frequencies. Examples of light energy include visible light, infrared radiation, and ultraviolet radiation.

As used herein, the term "lipid" refers to a variety of compounds that are characterized by their solubility in organic solvents. Such compounds include, but are not limited to, fats, waxes, steroids, sterols, glycolipids, glycosphingolipids (including gangliosides), phospholipids, terpenes, fat-soluble vitamins, prostaglandins, carotenes, and chlorophylls. As used herein, the phrase "lipid-based materials" refers to any material that contains lipids.

As used herein, "nucleic acid," "nucleotide sequence," or "nucleic acid sequence" refers to a nucleotide, oligonucleotide, polynucleotide, or any fragment thereof and to naturally occurring or synthetic molecules, such as L-DNA, phosphorothioates, locked nucleic acids, *etc.*

As used herein, the terms "pasteurize" and "pasteurization" refer to treatment processes where materials are heated, to temperatures and for periods of time sufficient to at least partially sterilize the material against microbial and/or mold growth. Pasteurized materials are characterized by prolonged stability against spoilage by microbial and mold growth. The terms "pasteurize" and "pasteurization" includes the more restrictive terms "sterilize" and "sterilization" where the treated material is substantially free of microbial and mold growth. Pasteurization typically uses temperatures below the boiling point of water.

As used herein, the term "physiological conditions" refers to temperature, pH, ionic strength, viscosity, and like biochemical parameters which are compatible with a viable organism, and/or which typically exist intracellularly in a viable mammalian cell.

The terms "polypeptide," "protein," and "peptide" are used herein interchangeably to refer to amino acid chains in which the amino acid residues are linked by peptide bonds or modified peptide bonds. The amino acid chains can be of any length of greater than two amino acids. Most proteins fold into unique three-dimensional structures. The shape into which a protein naturally folds is known as its native conformation. Although many proteins can fold unassisted, simply through the chemical properties of their amino acids, others require the aid of molecular chaperones to fold into their native states. Biochemists often refer to four distinct aspects of a protein's structure. The "primary structure" refers to the amino acid sequence. The "secondary structure" refers to regularly repeating local structures stabilized by hydrogen bonds. The most common examples are the alpha helix, beta sheet and turns. Because secondary structures are local, many regions of different secondary structure can be present in the same protein molecule. The "tertiary structure" refers to the overall shape of a single protein molecule, *i.e.,* the spatial relationship of the secondary structures to one another. Tertiary structure is generally stabilized by non-local interactions, most commonly the formation of a hydrophobic core, but also through salt bridges, hydrogen bonds, disulfide bonds, and even post-translational modifications. The "quaternary structure" refers to the structure formed by several protein molecules (polypeptide chains), usually called protein subunits in this context, which function as a single protein complex.

As used herein, a "polysaccharide" is a polymer composed of monosaccharides linked to one another. In many polysaccharides, the basic building block of the polysaccharide is actually a disaccharide unit, which can be repeating or non-repeating. Thus, a unit when used with respect to a polysaccharide refers to a basic building block of a polysaccharide and can include a monomeric building block (monosaccharide) or a dimeric building block (disaccharide). The term polysaccharide is also intended to embrace an oligosaccharide. Polysaccharides include, but are not limited to, glycosaminoglycans such as chondroitin sulfate, dermatan sulfate, heparin, heparin-like glycosaminoglycans (HLGAGs), heparan sulfate, hyaluronic acid, keratan sulfate, chitin, and derivatives and analogs thereof.

The term "stabilizing" or "stabilize" refers to a process that improves or maintains the structure or conformation of compositions containing labile biomolecules described herein, including, for example, proteins, nucleic acids, polysaccharides, or the like. The improved stability involves, for example, increased resistance of the biomolecules against destruction, decomposition, degradation, denaturation, aggregation and the like. The term "at least partially stabilize" as used herein means greater than about 5%, greater than about 10%, greater than about 20%, greater than about 30%, greater than about 40%, greater than about 50%, greater than about greater than about 60%, greater than about 70%, or greater than about 80% of the labile biomolecules in an aqueous solution are maintained in a native structural conformation, *e.g.*, an active conformation, when exposed to light energy as described herein, compared to an equivalent sample of a labile biomolecule that is not exposed to light energy.

### Apparatuses and Methods

The apparatuses and methods described herein are based on the discovery that irradiation of biomolecules with a low power light energy can maintain the folded state of proteins. The effect mimics the activity of protein chaperones.

Loss of activity of many biomolecules, such as proteins, is due to the loss of structural integrity caused by unfolding. Many protein-containing drugs (*e.g.*, protein biologics or vaccines) and protein-rich food stuffs lose functional properties upon heating or storage due to misfolding of the proteins that is often followed by irreversible formation of protein aggregates. This disclosure relates generally to methods for stabilizing biomolecules in an aqueous solution by exposing the biomolecules to light energy. For example, the biomolecules can be a polypeptide, a lipid, a nucleic acid, or a polysaccharide. The biomolecules may be contained in a material such as a food, a drink, a therapeutic agent (such as a biologic drug or vaccine), or a medical device (such as an implant). The radiation acts as a "chaperone" that assists folding of proteins or prevents unfolding of the same. Thus, the methods described herein can prevent the degradation of biomolecules during storage and processing.

In some embodiments, low power red laser irradiation is used to prevent loss of structural integrity (*i.e.*, denaturation) of labile biomolecules. In one embodiment, stabilization of biomolecules can be obtained by exposing low intensity red laser radiation to a material.

In some embodiments, stabilization can be induced by laser radiation operating at a wavelength from about 550 nm to about 750 nm, from about 575 nm to about 725 nm, from about 600 to about 700 nm, or from about 600 to about 650 nm. In an illustrative embodiment, stabilization can be induced by laser radiation operating at a wavelength of about 630 ± 10 nm.

Stabilization can be induced by low intensity red laser radiation having a power density less than 1.8 watt/cm², or less than about 1.5 watt/cm². In some embodiments, stabilization can be induced by low intensity red laser radiation having a power density at least about 0.1 watt/cm², at least about 0.2 watt/cm², at least about 0.3 watt/cm², at least about 0.4 watt/cm², at least about 0.5 watt/cm², or at least about 0.6 watt/cm² In a particular embodiment, the intensity of the red laser radiation may be about 0.63 watt/cm². While the embodiments herein are not limited to the use of laser, the light energy induced stabilization of labile biomolecules appears to be most pronounced using laser, which may be due to coherent simultaneous excitation of several molecules, such as water nanoclusters.

In an illustrative embodiment, the low intensity red laser can be a small low power visible, continuous helium-neon laser, such as that made by US Laser Corporation. This laser operates at about 632.8 nm, in the red portion of the spectrum. The low intensity red laser can also have a polarized output. For example, the output beam diameter of the laser can be less than about 1 mm.

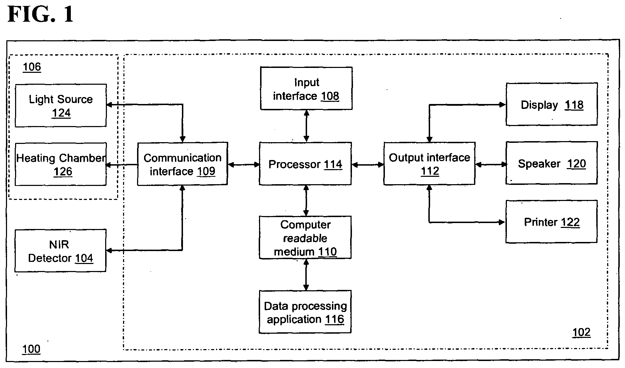

With reference to FIG. 1, a block diagram of a system for stabilizing labile biomolecules 100 is shown in accordance with an illustrative embodiment. Biomolecule stabilization system 100 may include a computing system 102, a NIR detector 104, and an biomolecule stabilization chamber 106. Different and additional components may be incorporated into biomolecule stabilization system 100. Computing system 102 may include an input interface 108, a communication interface 109, a computer-readable medium 110, an output interface 112, a processor 114, a data processing application 116, a display 118, a speaker 120, and a printer 122. Different and additional components may be incorporated into computing system 102.

Input interface 108 provides an interface for receiving information from the user for entry into computing system 102 as known to those skilled in the art. Input interface 108 may use various input technologies including, but not limited to, a keyboard, a pen and touch screen, a mouse, a track ball, a touch screen, a keypad, one or more buttons, etc. to allow the user to enter information into computing system 102 or to make selections presented in a user interface displayed on display 118. The same interface may support both input interface 108 and output interface 112. For example, a touch screen both allows user input and presents output to the user. Computing system 102 may have one or more input interfaces that use the same or a different input interface technology.

Communication interface 109 provides an interface for receiving and transmitting data between devices using various protocols, transmission technologies, and media as known to those skilled in the art. Communication interface 109 may support communication using various transmission media that may be wired or wireless. Computing system 102 may have one or more communication interfaces that use the same or a different communication interface technology. Data and messages may be transferred between computing system 102, fluorescence detector 104, and/or biomolecule stabilization chamber 106 using communication interface 109.

Computer-readable medium 110 is an electronic holding place or storage for information so that the information can be accessed by processor 114 as known to those skilled in the art. Computer-readable medium 110 can include, but is not limited to, any type of random access memory (RAM), any type of read only memory (ROM), any type of flash memory, etc. such as magnetic storage devices (e.g., hard disk, floppy disk, magnetic strips, ...), optical disks (e.g., CD, DVD, ...), smart cards, flash memory devices, etc. Computing system 102 may have one or more computer-readable media that use the same or a different memory media technology. Computing system 102 also may have one or more drives that support the loading of a memory media such as a CD or DVD. Computer-readable medium 110 may provide the electronic storage medium for fluorescence detector 104 and/or biomolecule stabilization chamber 106. Computer-readable medium 110 further may be accessible to computing system 102 through communication interface 109.

Output interface 112 provides an interface for outputting information for review by a user of computing system 102. For example, output interface 112 may include an interface to display 118, speaker 120, printer 122, etc. Display 118 may be a thin film transistor display, a light emitting diode display, a liquid crystal display, or any of a variety of different displays known to those skilled in the art. Speaker 120 may be any of a variety of speakers as known to those skilled in the art. Printer 122 may be any of a variety of printers as known to those skilled in the art. Computing system 102 may have one or more output interfaces that use the same or a different interface technology. Display 118, speaker 120, and/or printer 122 further may be accessible to computing system 102 through communication interface 109.

Processor 114 executes instructions as known to those skilled in the art. The instructions may be carried out by a special purpose computer, logic circuits, or hardware circuits. Thus, processor 114 may be implemented in hardware, firmware, or any combination of these methods and/or in combination with software. The term "execution" is the process of running an application or the carrying out of the operation called for by an instruction. The instructions may be written using one or more programming language, scripting language, assembly language, etc. Processor 114 executes an instruction, meaning that it performs/controls the operations called for by that instruction. Processor 114 operably couples with input interface 108, with communication interface 109, with computer-readable medium 110, and with output interface 112, to receive, to send, and to process information. Processor 114 may retrieve a set of instructions from a permanent memory device and copy the instructions in an executable form to a temporary memory device that is generally some form of RAM. Computing system 102 may include a plurality of processors that use the same or a different processing technology.

Data processing application 116 performs operations associated with processing data for a sample gathered using one or more electronic devices that continuously, periodically, and/or upon request monitor, sense, measure, etc. the physical and/or chemical characteristics of the sample. The operations may be implemented using hardware, firmware, software, or any combination of these methods. With reference to the illustrative embodiment of FIG. 1, data processing application 116 is implemented in software (comprised of computer-readable and/or computer-executable instructions) stored in computer-readable medium 110 and accessible by processor 114 for execution of the instructions that embody the operations of data processing application 116. Data processing application 116 may be written using one or more programming languages, assembly languages, scripting languages, etc.

NIR detector 104 may include a fluorescence detection system such as a fluorometer, etc. NIR detector 104 generates data related to a sample, such as the aggregation of the biomolecules in the sample. The source of and the dimensionality of the data is not intended to be limiting. Computing system 102 may be separate from or integrated with NIR detector 104 to control the operation of NIR detector 104.

Biomolecule stabilization chamber 106 may include an light source 124 and, optionally, a heating chamber 126. Different and additional components may be incorporated into biomolecule stabilization chamber 106. Light source 124 produces sufficient light energy to stabilize the biomolecules. Heating chamber 126 produces sufficient thermal energy to pasteurize the sample within the biomolecule stabilization chamber 106.

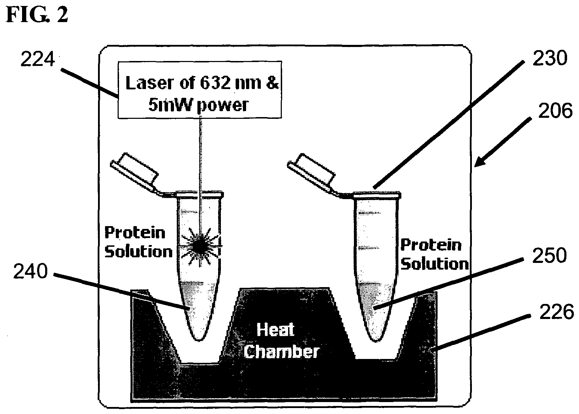

FIG. 2 shows an illustrative apparatus for testing the effects of light energy on the aggregation of a biomolecule in aqueous solution. The biomolecule stabilization chamber 206 includes a heating chamber 226 for holding a sample vial 230. At least one sample vial 230 contains a test biomolecule sample 240 that needs to be at least partially stabilized during a treatment in the chamber 206. The test biomolecule sample 240 is exposed to a light source 224 during the treatment of the test biomolecule sample 240. The treatment can be pasteurization, for example. Optionally, at least one sample holder contains a reference biomolecule sample 250 that is not exposed to light energy, but is otherwise exposed to the same treatment as that of the test biomolecule sample 240 in the apparatus of FIG. 2.

The light source 224 can be included within the apparatus of FIG. 2 or can be external to the apparatus and directed to the test biomolecule sample 240. The light source 224 is selected to have the wavelength and power to produce a stabilizing effect on the test biomolecule sample 240. For example, in an illustrative embodiment of the light source 224 of FIG. 2, light source 224 is made by US Laser Corporation, operating at 632.8 nm, in the red portion of the spectrum, with a power density of 0.63 Watt/cm².

In another embodiment, the apparatus can be a continuous flow treatment device wherein the test sample is exposed to light energy such as laser for a given period of time while the sample is resident in the continuous flow treatment device. For example, the continuation flow treatment device can be plug flow reactor type device or a tube and shell heat exchange type device. The test sample in the continuous flow treatment device can be made to flow through tubes or channels while being treated, for example heat treated for pasteurization, while also being exposed to light energy for stabilization.

In one embodiment, the apparatus of FIG. 2 can have a controller (not shown) configured to control the operating wavelength and power density of the light energy. The controller can be a feedback controller to control a power density of the light energy based on the signal detected by a detector (not shown).

In one aspect, the present disclosure relates to methods for stabilizing labile biomolecules in an aqueous solution and preventing denaturation. Denaturation is commonly defined as any covalent or non-covalent change in the structure of a biomolecule. In the case of proteins, this change may alter the secondary, tertiary or quaternary structure of the molecules. The present methods may be applied to a wide variety of proteins and do not depend on particular prosthetic groups or a particular primary, secondary or tertiary structure. As detailed in the Examples, a number of proteins that have vastly different structures and characteristics have been tested. The use of light energy to induce stability of biomolecules in aqueous solution is considered to be applicable to many different types of biomolecules in water.

The denaturation of biomolecules can be measured in a variety of ways. One method utilized to follow the course of denaturation is to measure changes in solubility. For example, proteins vary greatly in their resistance to aggregation. The loss of solubility is one of a series of changes in structure that may occur in biomolecules in response to heat. In some embodiments, the methods of the present disclosure prevent a loss of solubility of the biomolecules in an aqueous solution. For example, labile biomolecules in an aqueous solution that were exposed to an effective amount of light energy may retain at least about 99% solubility; at least about 95% solubility; at least about 90% solubility; at least about 75% solubility; at least about 50% solubility; at least about 25% solubility; or at least about 10% solubility compared to a sample of the biomolecule that was not exposed to light energy.

For those proteins that are enzymes, denaturation can include the loss of structure, which renders the enzyme inactive. Changes in the rate of the reaction, the affinity for substrate, pH optimum, temperature optimum, specificity of reaction, etc., may be affected by denaturation of enzyme molecules. Loss of enzymatic activity can be a very sensitive measure of denaturation as some assay procedures are capable of detecting very low levels of product. In some cases, the loss of activity can be shown to occur only after some other changes in structure can be observed by other procedures. A number of protein molecules may exhibit biological activities that are not enzymatic in nature. Antibodies for instance are capable of interacting with specific antigen molecules. Other proteins, like hemoglobin, may function as carriers while some, *e.g.*, ferritin, may function in the storage of specific components. The loss of any of these activities can be measured as protein denaturation. In some embodiment, for those proteins that are enzymes, denaturation can be defined as the loss of enough structure to render the enzyme inactive, or to cause changes in the rate of the reaction, the affinity for substrate, pH optimum, temperature optimum, specificity of reaction, etc.

In some embodiments, the methods of the present disclosure prevent a loss of activity of the biomolecules in an aqueous solution. For example, labile biomolecules in an aqueous solution that were exposed to an effective amount of light energy may retain at least about 99% activity; at least about 95% activity; at least about 90% activity; at least about 75% activity; at least about 50% activity; at least about 25% activity; or at least about 10% activity compared to a sample of the biomolecule that was not exposed to light energy.

### Applications of the Present Methods

The biomolecules intended for use in the present methods include any biomolecules that are biologically or industrially useful. For example, a biologically useful biomolecule may be any biomolecule which can be employed in the diagnosis, cure, mitigation, treatment or prevention of disease or in the enhancement of desirable physical or mental development and conditions in man or animals. Polypeptides, especially proteins, for use in human and/or veterinary medicine or in diagnosis (*in vivo* or *in vitro*) are of particular interest. Industrially useful polypeptides are those employed analytically, in production or which are otherwise useful in the chemical industry. Biomolecules important for human and animal nutrition are also included.

In one aspect, the methods are used to stabilize one or more biomolecules present in drug products or medical devices during manufacturing or storage. The term "drug" refers to any substance that, when absorbed into the body of a living organism, alters normal bodily function. Drugs include toxins, food additives, allergens, supplements, vitamins, among others. Thus, uses for some of the methods described herein include improving the stability of therapeutic polypeptides during manufacturing and storage. Such polypeptides may be used, for example, in immunization (as vaccines), *in vitro* or *in vivo* diagnostics (to increase the solubility/stability of antigens or antibodies), and therapeutically useful polypeptides such as growth factors, hormones and like bioactive peptides, as illustrated by α-1-antitrypsin, atrial natriuretic factor (diuretic), calcitonin, calmodulin, choriogonadotropin (α and β), colony stimulating factor, corticotropin releasing factor, β-endorphin, endothelial cell growth supplement, epidermal growth factor, erythropoietin, fibroblast growth factor, fibronectin, follicle stimulating hormone, granulocyte colony stimulating factor, growth hormone (somatotropin), growth hormone releasing factor (somatoliberin), insulin, insulin-like growth factor (somatomedin), an interferon, an interleukin, lutropin, lymphotoxin, macrophage derived growth factor, macrophage inhibiting factor, macrophage stimulating factor, megakaryocyte stimulating factor, nerve growth factor, pancreatic endorphin, parathyroid hormone, platelet derived growth factor, relaxin, secretin, skeletal growth factor, superoxide dismutase, thymic hormone factor, thymic factor, thymopoietin, thyrotropin, tissue plasminogen activator, transforming growth factor (α and β), tumor necrosis factor, tumor angiogenesis factor, vasoactive intestinal polypeptide and wound angiogenesis factor; immunosuppressives, such as RHO (D) ISG and IVGG's; thrombolytics such as urokinase, streptokinase and tissue plasminogen activator; and antigens such as Rhus all (poison ivy), Rhus tox poison ivy-polyvalent and staphage lysate (staphylococcus lysate).

In one aspect, the methods are used to stabilize protein-containing drugs (*e.g.*, vaccines), which can lose functional properties during manufacturing and storage. Storage and thermal treatment of such substances can therefore be enhanced using the stabilization methods using light energy described herein. One application can be to store labile biomolecules, where an aqueous solution of the protein is stored under constant light energy.

In one aspect, the methods are used to stabilize one or more biomolecules present in food and beverage products during processing, such as pasteurization. Foodstuffs like milk and eggs need to be pasteurized by thermal treatment in order to be free of pathogenic organisms. While high temperature pasteurization is desirable from the point of killing certain undesirable organisms, pasteurization typically uses high temperatures in which proteins can irreversibly aggregate. The types of pasteurization processes include: High Temperature/Short Time (HTST), Extended Shelf Life (ESL) treatment, and Ultra-high temperature (UHT or ultra-heat treated). In the HTST process, a biomaterial such as milk is forced between metal plates or through pipes heated on the outside by hot water, and is heated to 71.7°C (161°F) for 15-20 seconds. UHT processing holds the biomaterial such as milk at a temperature of 138°C (280°F) for a fraction of a second. ESL treatment has a microbial filtration step and is done at lower temperatures than HTST.

### EXAMPLES

The present compositions, methods and kits, thus generally described, will be understood more readily by reference to the following examples, which are provided by way of illustration and are not intended to be limiting of the present methods and kits.

### Example 1 - Stabilization of Proteins Using Red Laser Light

In this Example, the effects of light energy on the aggregation of a labile biomolecule in aqueous solution were examined. The device included a heating element and a laser, as depicted in FIG. 2. The laser operated at 632.8 nm, in the red portion of the spectrum, with a power density of 0.63 watts/cm². The 180 degree scattering at 360 nm (in terms of OD at 360 nm) was taken to measure the extent of aggregation because it is well-known that this scattering is a measure of the propensity of a protein to form aggregate. Aggregation of proteins of the reference samples was taken as 100% and the aggregation of laser-treated proteins in the test samples was calculated relative to the 100% aggregation of the reference samples.

A variety of proteins were chosen, including hemoglobin, an alpha helix rich protein, which aggregates at 60°C; insulin, a very small protein that is aggregated at 40°C by a disulphide linkage breaker like dithiothreitol; and citrate synthase, a beta sheet-rich enzyme which readily aggregates by thermal treatment at 45°C.

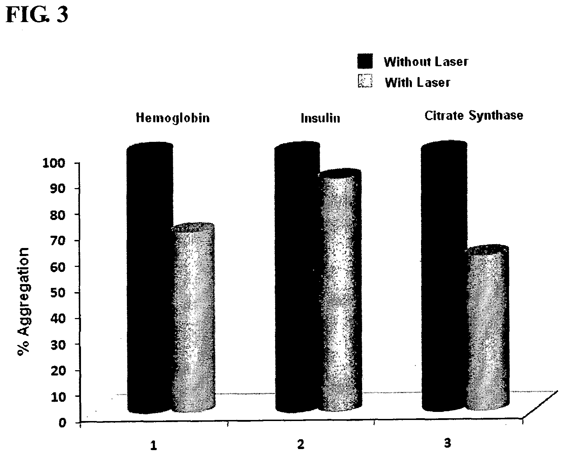

*Thermal aggregation of hemoglobin..* Hemoglobin was purchased from Sigma-Aldrich (St. Louis, MO) and a 0.5 mg/ml stock solution was prepared by mixing with 100 mM phosphate buffer solution of pH 7.2. The aggregation of a 0.1 mg/ml hemoglobin solution was observed by measuring the absorption of radiation of 360 nm wavelength with a time duration of 20 min in a Perkin Elmer spectrophotometer at 60°C. Test samples were irradiated with He-Ne laser having power density is 0.63 watts/cm² and operating at a wavelength 632.8 nm. A control sample was subjected to identical conditions expect laser irradiation was not performed. The results are shown in FIG. 3 and indicate that irradiation with laser light reduced the formation of hemoglobin aggregates by about 25% compared to an non-irradiated control sample.

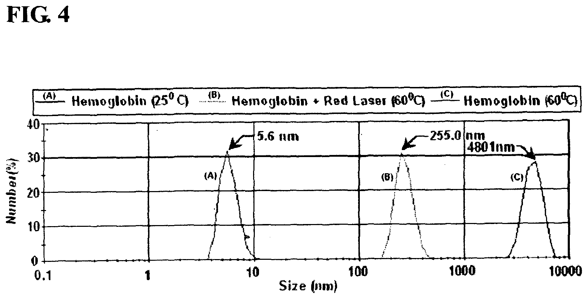

FIG. 4 represents the dynamic light scattering pattern of protein hemoglobin in the presence and absence of red laser light at 60°C. The hydrodynamic diameter of hemoglobin at 25°C is 5.6 nm. When this protein solution was heated at 60°C without red laser light for 20 min, the hydrodynamic diameter increases to 4801.0 nm. However, in the presence of red laser light the diameter increases to only 255.0 nm. Thus, these data confirm that irradiation with the laser prevented aggregation of hemoglobin.

*Dithiothreitol induced aggregation of insulin.* Insulin was purchased from ICN and a stock solution of insulin (0.5 mg/ml) was prepared by mixing with 100 mM phosphate buffer of pH 7.2. Disulphide linkages of insulin (0.1 mg/ml) were reduced by dithiothreitol (DTT, 20 mM), which leads aggregation of insulin at 40°C. This aggregation of protein was measured by absorption at 360 nm after heat treatment at 40°C in a Perkin Elmer spectrophotometer. Aggregation of insulin was measured in the presence and in the absence of laser light. Test samples were irradiated with a He-Ne laser having power density is 0.63 watts/cm² and operating at a wavelength 632.8 nm. A control sample was subjected to identical conditions expect that laser irradiation was not performed. The results are shown in FIG. 3 and indicate that irradiation with laser light reduced the formation of insulin aggregates by about 10% compared to a non-irradiated control sample.

*Thermal Aggregation of Citrate Synthase.* Citrate synthase was purchased from Sigma-Aldrich and a 0.5 mg/ml stock solution was prepared by mixing with 100 mM phosphate buffer solution of pH 7.2. Citrate synthase was readily converted to its aggregated state from a native state at 45°C. The aggregation profile of the protein (0.1 mg/ml) was measured by measuring optical density (OD) at 360 nm in a Perkin Elmer spectrophotometer in 100 mM phosphate buffer of pH 7.2. Test samples were irradiated with a He-Ne laser having power density is 0.63 Watt/cm² and operating at a wavelength 632.8 nm. A control sample was subjected to identical conditions expect that laser irradiation was not performed. The results are shown in FIG. 3 and indicate that irradiation with laser light reduced the formation of protein aggregates by about 40% compared to an non-irradiated control sample.

It is thus clear that irradiation of the a protein-containing sample with laser light leads to prevention of aggregation of proteins. Such prevention of aggregation mimics a chaperone-assisted folding. In this case, the ordered state of water molecules may provide a hydrophobic template to maintain the integrity of the proteins. As such, the present methods are useful for reducing aggregation of thermally labile biomolecules.

### Example 3- Comparison of the Effects of Red Laser Power on Aggregation of Citrate Synthase.

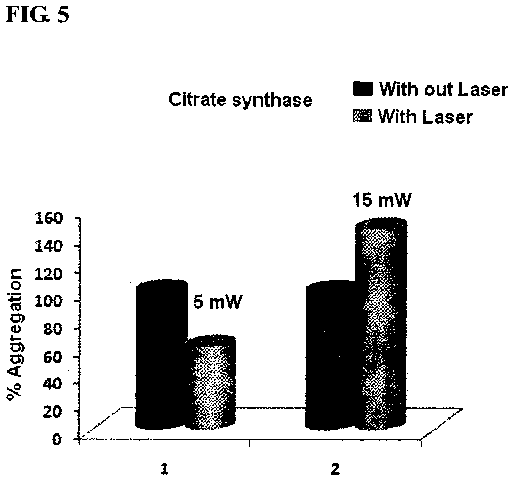

A comparative study showing the effect of the power of the red laser on the aggregation of citrate synthase was conducted. Thermal aggregation of citrate synthase was determined by measuring absorption of radiation of 360 nm wavelength at 45°C. Aggregation of control samples (in absence of laser treatment) was considered as 100% aggregation while aggregation of the treated protein of the test samples was calculated relative to the 100% aggregation of the control samples. FIG. 5 shows 60% aggregation in presence of a red laser operating at 632.8 nm, 5 mW power, with a power density of 0.63 watts/cm². However, when the power of the same red laser was increased by a factor of three to 15 mW, with a power density of 1.9 watts/cm², the aggregation of the test samples was approximately 145%. Thus, increasing the energy input to the test samples beyond that needed to increase the activation energy for unfolding the protein appears to generate heat energy that increases aggregation. As such; suitable methods for preventing protein aggregation use a low-power red laser.

## Claims

1. A method for stabilizing biomolecules comprising:
exposing one or more labile biomolecules in aqueous solution to an effective amount of light energy to at least partially stabilize the one or more labile biomolecules in the solution,
wherein the light energy has a wavelength of 630 ± 20 nm and a power density of less than 1.8 watts/cm²,

2. The method of claim 1, wherein the one or more labile biomolecules are exposed to the light energy in the presence of a denaturant.

3. The method of claim 2, wherein the denaturant is heat or a chemical denaturant.

4. The method of claim 3, wherein the heat denaturant includes heating to a temperature from about 40°C to about 100°C and the chemical denaturant is a reducing agent.

5. The method according to claim 1, wherein the power density is at least 0.1 watts/cm².

6. The method of any of claims 1-5, wherein the one or more labile biomolecules are selected from the group consisting of: proteins, nucleic acids, lipids, and polysaccharides.

7. The method of claim 6, wherein the one or more labile biomolecules are proteins.

8. The method of claim 7, wherein the proteins undergo aggregation and/or unfolding in the absence of the effective amount of light energy.

9. The method of claim 8, wherein aggregation of the proteins is reduced from about 10% to about 60% compared to the aggregation of the proteins not exposed to the light energy.

10. The method of claim 7, wherein the proteins are selected from the group consisting of: hemoglobin, insulin, and citrate synthase.

11. The method of any of claims 1-10, wherein the one or more labile biomolecules in aqueous solution are in a material selected from the group consisting of: a food, a drink, a therapeutic agent, an implant, and combinations thereof.

12. The method of claim 11, wherein the food is an egg preparation.

13. The method of claim 11, wherein the drink is milk.

14. The method of any of claims 1-13, wherein the light energy is red wavelength laser radiation.

15. The method of any of claims 1-14, wherein the light energy produces a fluorescence emission at 900 ± 10 nm from water.

16. The method of any of claims 1-15 further comprising simultaneously pasteurizing the material.

17. The method of claim 16, wherein the material comprises an industrial enzyme, a recombinant protein, a food, a drink, a therapeutic agent, and medical device.

## Patentansprüche

1. Verfahren zum Stabilisieren von Biomolekülen, das umfasst:
Aussetzen eines oder mehrerer labiler Biomoleküle in wässriger Lösung einer wirksamen Menge von Lichtenergie, um das eine oder die mehreren labilen Biomoleküle in der Lösung wenigstens teilweise zu stabilisieren,
wobei die Lichtenergie eine Wellenlänge von 630 ± 20 nm und eine Energiedichte von weniger als etwa 1,8 Watt/cm² hat.

2. Verfahren nach Anspruch 1, wobei das eine oder die mehreren labilen Biomoleküle der Lichtenergie in Gegenwart eines Denaturierungsmittels ausgesetzt werden.

3. Verfahren nach Anspruch 2, wobei das Denaturierungsmittel Wärme oder ein chemisches Denaturierungsmittel ist.

4. Verfahren nach Anspruch 3, wobei das Wärmedenaturierungsmittel das Erwärmen auf eine Temperatur von etwa 40°C bis etwa 100°C umfasst und das chemische Denaturierungsmittel ein Reduktionsmittel ist.

5. Verfahren nach Anspruch 1, wobei die Energiedichte wenigstens 0,1 Watt/cm² beträgt.

6. Verfahren nach einem der Ansprüche 1 - 5, wobei das eine oder die mehreren labilen Biomoleküle aus der Gruppe ausgewählt werden, die aus Proteinen, Nukleinsäuren, Lipiden und Polysacchariden besteht.

7. Verfahren nach Anspruch 6, wobei das eine oder die mehreren labilen Biomoleküle Proteine sind.

8. Verfahren nach Anspruch 7, wobei die Proteine in Abwesenheit einer wirksamen Menge von Lichtenergie einer Aggregation und/oder Entfaltung unterzogen werden.

9. Verfahren nach Anspruch 8, wobei die Aggregation der Proteine von etwa 10% auf etwa 60% im Vergleich mit der Aggregation der Proteine reduziert wird, die nicht der Lichtenergie ausgesetzt sind.

10. Verfahren nach Anspruch 7, wobei die Proteine aus der Gruppe ausgewählt werden, die aus Hämoglobin, Insulin und Citratsynthase besteht.

11. Verfahren nach einem der Ansprüche 1 - 10, wobei das eine oder die mehreren labilen Biomoleküle in wässriger Lösung in einem Material vorkommen, das aus der Gruppe ausgewählt wird, die aus einem Nahrungsmittel, einem Getränk, einem therapeutischen Mittel, einem Implantat und Kombinationen davon besteht.

12. Verfahren nach Anspruch 11, wobei das Nahrungsmittel eine Eierzubereitung ist.

13. Verfahren nach Anspruch 11, wobei das Getränk Milch ist.

14. Verfahren nach einem der Ansprüche 1 - 13, wobei die Lichtenergie eine Laserstrahlung mit roter Wellenlänge ist.

15. Verfahren nach einem der Ansprüche 1 - 14, wobei die Lichtenergie eine Fluoreszenzemission bei 900 ± 10 nm von Wasser erzeugt.

16. Verfahren nach einem der Ansprüche 1 - 15, das des Weiteren das gleichzeitige Pasteurisieren des Materials umfasst.

17. Verfahren nach Anspruch 16, wobei das Material ein industrielles Enzym, ein rekombinantes Protein, ein Nahrungsmittel, ein Getränk, ein therapeutisches Mittel und eine medizinische Vorrichtung umfasst.

## Revendications

1. Procédé de stabilisation de molécules biologiques comprenant :
l'exposition d'une ou plusieurs molécules biologiques labiles en solution aqueuse à une quantité efficace d'énergie lumineuse afin de stabiliser au moins partiellement lesdites molécules biologiques labiles dans la solution,
où l'énergie lumineuse présente une longueur d'onde de 630 ± 20 nm et une densité d'énergie inférieure à 1,8 watt/cm².

2. Procédé selon la revendication 1, dans lequel la une ou plusieurs molécules biologiques labiles sont exposées à l'énergie lumineuse en présence d'un agent de dénaturation.

3. Procédé selon la revendication 2, dans lequel l'agent de dénaturation est la chaleur ou un agent de dénaturation chimique.

4. Procédé selon la revendication 3, dans lequel l'agent de dénaturation chaleur comprend le chauffage à une température d'environ 40°C à environ 100°C et l'agent de dénaturation chimique est un agent réducteur.

5. Procédé selon la revendication 1, dans lequel la densité d'énergie est d'au moins 0,1 watt/cm².

6. Procédé selon l'une quelconque des revendications 1 à 5, dans lequel la une ou plusieurs molécules biologiques labiles sont sélectionnées dans le groupe constitué des : protéines, acides nucléiques, lipides, et polysaccharides.

7. Procédé selon la revendication 6, dans lequel la une ou plusieurs molécules biologiques labiles sont des protéines.

8. Procédé selon la revendication 7, dans lequel les protéines subissent l'agrégation et/ou le dépliement en l'absence de la quantité efficace d'énergie lumineuse.

9. Procédé selon la revendication 8, dans lequel l'agrégation des protéines est réduite d'environ 10 % à environ 60 % comparée à l'agrégation des protéines non exposées à l'énergie lumineuse.

10. Procédé selon la revendication 7, dans lequel les protéines sont sélectionnées dans le groupe constitué : de l'hémoglobine, de l'insuline, et de la citrate synthase.

11. Procédé selon l'une quelconque des revendications 1 à 10, dans lequel la une ou plusieurs molécules biologiques labiles en solution aqueuse se trouvent dans un matériau sélectionné dans le groupe constitué : d'un aliment, d'une boisson, d'un agent thérapeutique, d'un implant, et de leurs combinaisons.

12. Procédé selon la revendication 11, dans lequel l'aliment est une préparation à base d'oeuf.

13. Procédé selon la revendication 11, dans lequel la boisson est le lait.

14. Procédé selon l'une quelconque des revendications 1 à 13, dans lequel l'énergie lumineuse est le rayonnement laser de longueur d'onde rouge.

15. Procédé selon l'une quelconque des revendications 1 à 14, dans lequel l'énergie lumineuse produit une émission de fluorescence à 900 ± 10 nm à partir de l'eau.

16. Procédé selon l'une quelconque des revendications 1 à 15 comprenant en outre simultanément la pasteurisation du matériau.

17. Procédé selon la revendication 16, dans lequel le matériau comprend une enzyme industrielle, une protéine recombinante, un aliment, une boisson, un agent thérapeutique, et un dispositif médical.
